**Europäisches Patentamt**

**European Patent Office**

**Office européen des brevets**

(19)

(11)  **EP 0 859 654 B1**

(12)  **EUROPEAN PATENT SPECIFICATION**

(45) Date of publication and mention
of the grant of the patent:
**08.12.1999 Bulletin 1999/49**

(51) Int. Cl.$^6$: **B01D 3/42**, G01N 27/04,
G01N 33/483, G05D 21/00,
C02F 1/04

(21) Application number: **96931758.5**

(22) Date of filing: **20.09.1996**

(86) International application number:
**PCT/DK96/00400**

(87) International publication number:
**WO 97/10884 (27.03.1997 Gazette 1997/14)**

(54) **A METHOD FOR ADJUSTING A SEPARATION PROCESS AND AN APPARATUS FOR USE OF THE METHOD**

METHODE ZUR ANPASSUNG EINES TRENNPROZESSES UND APPARAT ZUR ANWENDUNG DERSELBEN

PROCEDE D'ADAPTATION POUR TECHNIQUE DE SEPARATION ET APPAREIL EMPLOYE AVEC CE PROCEDE

(84) Designated Contracting States:
**DE ES FR GB IE NL**

(30) Priority: **22.09.1995 DK 105795**

(43) Date of publication of application:
**26.08.1998 Bulletin 1998/35**

(73) Proprietor: **Agro Miljo A/S**
**6400 Sonderborg (DK)**

(72) Inventor:
**BASTHOLM, Jeppe, Christian**
**DK-6400 Soenderborg (DK)**

(74) Representative: **Nielsen, Leif**
**Patrade A/S**
**Aaboulevarden 21**
**8000 Aarhus C (DK)**

(56) References cited:
**WO-A-96/03191**

• **DIALOG INFORMATION SERVICES, File 350, Derwent World Patent, Dialog Accession No. 001148678, WPI Accession No. 74-22420V/12, SEVZAPMONTAZHAVTOMATIKA et al., "Urea Production- by Reaction of Acetic Acid and Ammonia, Separation Product and Removal of Ammonia by Scrubbing and Distillation"; & SU,A,386 930, 03-10-73, 7412, (Basic).**

## Description

The present invention relates to a method for adjusting a separation process in cleaning of waste water, preferably liquid manure, and wherein the concentrations of ammonia and acetic acid in the water fraction from the separation process are determined, and wherein said concentrations are used as parameters for adjusting the separation process so that the concentrations of ammonia and acetic acid are kept within predetermined limits. The invention further relates to an apparatus for use by the method.

The method may also be applied to other waste water from biological processes, e.g. in the pharmaceutical industry or cornflour production.

In the separation of manure it is known to perform a measurement of the contents of ammonia and acetic acid in the water fraction. These measurements are performed in order to add chemicals, which are brought to react with ammonia ad acetic acid in order to bring the concentration of these elements within permitted limits. Such limits will normally be the limits that are determined in advance by public authority requirements regarding the purity of water let to a recipient.

By the known methods a measurement is performed directly of ammonia and acetic acid. However, such measurements are inconvenient as they involve a time lag for laboratory measurements to provide an exact result that may be used in the adjustment of the process.

If an exact measurement of the concentration of ammonia and acetic acid is not performed, a subsequent addition of acid and base in order to precipitate these two elements will either cause an overdosing or an underdosing of the chemicals. In either case the quality of the outlet water will be affected negatively and may make the outlet to the recipient impossible. Thus, in case of overdimensioning the outlet water may become either alkaline or acid. In case of underdimensioning insufficient amounts are neutralised for the concentration of ammonia and/or acetic acid to be kept within the predetermined limits.

The known methods are furthermore based on a measurement read by a user. Then the user adds acid and/or base according to a calculation model in order to obtain the desired neutralisation. Thus, the known methods are not suited for use in continuous operation with automatic addition of acid and base as a consequence of measured results.

It is the object of the present invention to provide a method that makes it possible to determine the concentration of ammonia and acetic acid in a reliable and efficient manner, and which at the same time allows automatic adjustment of the addition of reacting acid and base in order to neutralise ammonia and acetic acid. It is furthermore the object of the invention to provide an apparatus for use by the method.

This is obtained according to the present invention by a method characterised in that a distillation is part of the separation process, that the concentrations are determined in the water fraction from the distillation step by measuring values of pH and conductivity in the distillate, that said values are converted into equivalent values of ammonia and acetic acid concentrations in the water fraction, and that these calculated values are used as parameters for controlling the addition of acid and base, which are reacted with ammonia and acetic acid for the neutralisation thereof.

It has turned out that the quality of the purified water expressed as an equivalent concentration of ammonia and acetic acid is an exact indicator of the water quality. Since a distillation is part of the separation process, the water fraction will only contain ions which cause a change of pH but no salts, which ordinary water will contain.

The method of measuring values of pH and conductivity and then converting these into the equivalent concentrations of ammonia and acetic acid will consequently be very reliable. The method of measuring may be used directly with adjustment of the separation process, and this adjustment may take place continuously and automatically.

Alternatively, it is also possible to use the calculated equivalent values for a manual control of the addition of acid and base if this is desired.

Irrespective whether the addition takes place automatically or manually as a result of the calculated equivalent values, it will be possible to add an exact amount of acid and base so that all ammonia and acetic acid is precipitated and without overdosing of acid and/or base taking place. Overdosing is an excessive resource consumption.

The method is particularly suited for a separation process in which mechanical vapour compression is involved. Such a process is described in International Patent Application No. PCT/DK95/00310. The method is used for controlling acid/base addition to a scrubber.

The method of measuring may also be applied to other separation processes as long as a distillation step is part thereof. The distillation step ensures that in the water only ions are present that cause a change of pH. If there were no distillation, then salts, including metal salts of sodium, magnesium, potassium ad calcium, would cause a change of pH to the alkaline side. This would not be distinguishable from the change resulting from ammonia and consequently would lead to a misdosing.

In the method of measuring according to the invention the kinds of fatty acids involved are not taken account of, as all acid components are considered acetic acids and, likewise, all base components are considered ammonia. In practice this has turned out to yield a correct measurement and correct control for neutralisations as the acetic acid constitutes 90% of all fatty acids in common biological waste water.

In a preferred separation process in which the method according to the invention is applied, manure is heated to the boiling point. The gas resulting from this is caused to condense under slight pressure increase in a heat exchanger. The energy that has to be taken from the vapour in order to cause it to condense is used for making the manure boil. This is the basic process in the so-called vapour compression principle.

According to a preferred embodiment the equivalent values of ammonia and acetic acid concentrations in the water fraction are calculated according to the following algorithms:

$$C_{NH_4} = \frac{S \cdot 10^{-pH} \, (\lambda_{H_3O^+} + \lambda_{CH_3COO^-}) - 10^{pH-14} \, (\lambda_{OH^-} \cdot \lambda_{CH_3COO^-})}{\left(1 - \dfrac{1}{1 + 10^{pKs\text{-}NH4\text{-}pH}}\right) \, \left(\lambda_{NH_4^+} + \lambda_{CH_3COO^-}\right)}$$

$$C_{CH_3COOH} = \frac{S \cdot 10^{-pH} \, (\lambda_{H_3O^+} \cdot \lambda_{NH_4^+}) - 10^{pH-14} \, (\lambda_{OH^-} + \lambda_{NH_4^+})}{\left(\dfrac{1}{1 + 10^{pKs\text{-}CH_3COOH\text{-}pH}}\right) \, \left(\lambda_{CH_3COO^-} + \lambda_{NH_4^+}\right)}$$

wherein S is the conductivity and $\lambda$ is the molar conductivity of the individual ions. Specific values of the various ion concentrations and well-known values of the molar conductivity of such ions may be inserted into the algorithms. The values may be obtained from common reference works.

The apparatus used by the method comprises heating members for boiling the manure, cooling members for cooling and condensing the formed vapours, container members for receiving the condensed water fraction, said apparatus being characterised in comprising measuring as well as computing and control members for measuring conductivity and pH values in the water fraction as well as for converting the measured values into equivalent concentrations of ammonia and acetic acid, and dosing members for adding acid and base to the water fraction in a amount according to signals from the computing and control members.

The apparatus according to the invention may have any design as long as it is ensured that the manure will be boiled so that vapour formation occurs followed by condensation. A specific design of the apparatus may be the type described in International Patent Application No. PCT/DK95/00310 and provided with measuring as well as computing and control members and dosing members for the addition of acid and base.

An apparatus of this type is simple to manufacture and is used unproblematically to obtain a reliable separation process wherein it is possible to optimise the use of acid and base in order to neutralise ammonia and acetic acid.

The apparatus is advantageously designed as a plant for mechanical vapour compression, the heating and cooling members being provided in the form of a heat exchanger arranged in a evaporation tank, which comprises a top and a sump interconnected by a tube comprising a circulation pump for fluid circulation, and a compressor being arranged between the sides of the heat exchanger to circulate the vapour, and said heat exchanger, circulation pump and compressor forming part of a mechanical vapour compression plant. It is further preferred for all components to be arranged in an insulated cabinet in order to minimise the energy consumption in the process.

The computing and control members may be provided with a display or a printer unit to show the signals if it is desired to use manually operated dosing members. Alternatively the apparatus may have computing and control members sending a signal that is used directly in automatic dosing members as a result of the incoming signal.

The invention will now be explained with reference to the accompanying drawing, wherein

Fig. 1     shows a schematic view of a apparatus for use by the method according to the invention, and
Fig. 2     a flowchart for illustration of the adjustment system according to the invention.

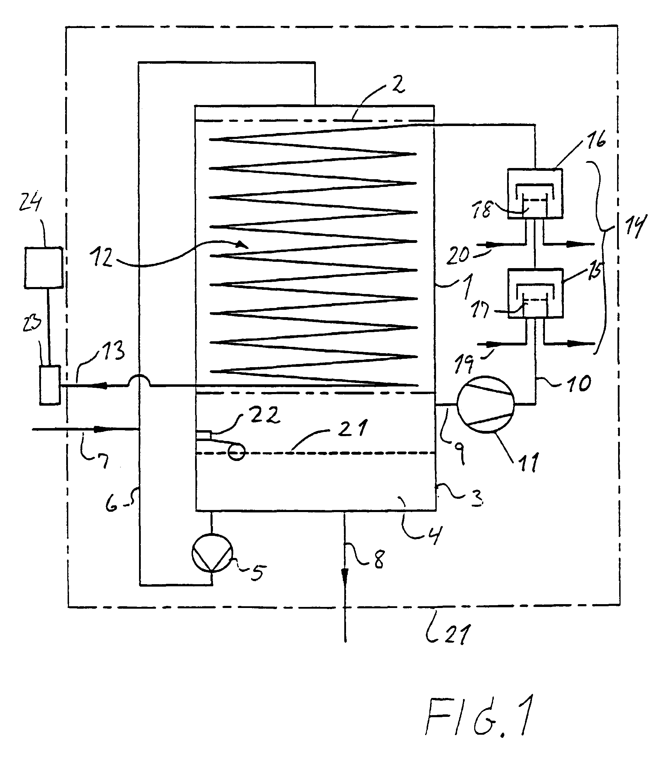

Figure 1 schematically illustrates a plant with an apparatus according to the invention. The plant is constructed for separation of polluted fluids by mechanical vapour compression. Thus, the plant is run according to a known principle for separating a polluted fluid part, preferably water, and concentrate the polluted part. The primary fluid part to be cleaned may consist of water but may also consist of other fluids such as freon polluted by oil.

The plant comprises an evaporator 1. At the top of the evaporator, a distributor system 2 is arranged, and at the bottom a vessel 3 containing the polluted fluid 4 is arranged. The vessel 3 is connected with a circulation pump 5 and a conduit 6 pumping the heated and polluted fluid 4 to the distributor system 2 in the evaporator top. The vessel 3 has a inlet 7 for feeding polluted fluid 4, and a discharge conduit 8 used for emptying the concentrated and polluted part out of the boiler 3.

At the top of the boiler 3 is a vapour outlet 9, which is connected via a conduit 10 and a compressor 11 to a heat exchanger 12 positioned in the evaporator 1. In the bottom of the heat exchanger 12 is a outlet 13 for condensate. A scrubber 14 is inserted in the conduit 10, where the compressor 11 is mounted, too. In the situation shown, the scrubber 14 is located upstream of the compressor 11. This is preferred, but it is also possible to position the scrubber 14 downstream of the compressor 11.

It should be noted that no construction of electric control of the plant is illustrated in the drawing. However, such a control system will be well-known to a person skilled in the art and, therefore, requires no detailed explanation.

Thus, the polluted fluid 4 is added in portions at the inlet 7 and let out via the discharge conduit 8 after a concentration has taken place. The condensate or distillate is removed via the outlet 13.

When the polluted fluid has been introduced into the vessel 3, the illustrated level 21 is obtained. This causes a level switch 22 to shift so that a heating member (not shown) and the circulation pump 5 are turned on. Thereafter, the temperature is brought to a temperature and pressure state lying immediately below the boiling point of the fluid to be cleaned (the condensate). Thus, in the case of water, the temperature is brought to nearly 100°C.

The circulation pump 5 is turned on when starting the plant in order to ensure that all components have the same temperature. When the temperature has reached approximately 100°C, the compressor 11 is turned on. The compressor 11 creates a low pressure in the vessel 3, thus forcing the vapour present over the polluted fluid 4 through the scrubber 14, whereupon the vapour is conducted via the conduit 10 to the compressor 11 and then into the heat exchanger 12, where there is a heat exchange of the vapour on one side of the heat exchanger and the heated polluted fluid 4 on the other side of the heat exchanger. This will cause the vapour having been compressed in the compressor 11 to deliver its energy, which is transferred to the circulated polluted fluid 4 on the other side of the heat exchanger. This will make the fluid to be cleaned evaporate. This vapour travels through the heat exchanger 12 via its first side and thus flows into the top of the vessel 3 and will flow via the vapour outlet 9 and through the scrubber 14, the conduit 10 and the compressor 11 into the heat exchanger 12. While delivering its energy, the vapour is condensed ad may subsequently be removed as condensate via the outlet 13.

The scrubber 14 comprises a first an a second scrubber 15, 16. The scrubber 15 contains an acid 17, and the scrubber 16 contains a base 18. Either scrubber 15, 16 is provided with an inlet line 19, 20 for feeding acid and base, respectively. Thus, it is possible on the basis of measurements to replace fluids 17, 18 so that the pH is kept substantially constant during evaporation. The scrubber 14 will preferably be arranged with the acid step 15 upstream of the alkaline step 16. This order is important as the acids are more volatile than the bases. In order to retain what remains in the latter step of the scrubber 14, this must be a base, e.g. sodium hydroxide.

The entire system is contained within a closed and insulated cabinet 21. In this manner a energy-neutral process is obtained as there is no interaction with the surroundings. Advantageously, this will also mean that the vapour is prevented from undesirable condensing in a step having a lower temperature. If there were "cold steps", the process would come to a halt as the vapour would just condense in such a cold step in stead of the desired condensing in the heat exchanger 12.

The scrubbers 15, 16 are provided with fillers to establish a large surface for reaction and to dampen the formation of bubbles, splashes and the like, which gives rise to sprays of fluid and, thus, the risk of drops being thrown out into the vapour conduit 10.

Measuring sondes 23 are arranged in the outlet 13 outside of the insulated cabinet 21. The measuring sondes 23 are connected to a computing and control unit 24. Said computing and control unit contains a microprocessor capable of converting the measured values into equivalent values of concentration of ammonia and acetic acid according to an algorithm stored therein, and these parameters may then be used to control chemical pumps for the addition of acid/base into the acid and alkaline steps 15, 16 of the scrubber 14.

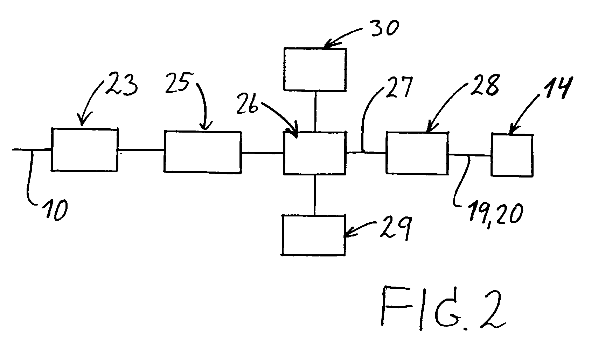

A flow chart of the measuring system is seen in Fig. 2. The measuring sondes 23 measuring on the conduit 10 are seen. The measuring sondes 23 send a signal via a galvanic separator 25 to a microprocessor 26. The galvanic separator has proven necessary to obtain measuring with sensitive sondes that are generally available in the market without their influencing each other galvanically when used in the same solution simultaneously. In the microprocessor a calculation is made and a signal is sent to chemical pumps 28 which feed acid and base via conduits 19, 20 to the scrubber 14. The microprocessor 26 is provided with a power supply 29 of its own so that it may be seen as a separate unit.

In the microprocessor 26 calculations are made of the equivalent concentrations of ammonia and acetic acid. Simultaneously, adjustment of the chemical addition is provided by means of the pumps 28. The adjustment parameters preferably used will be the addition of nitric acid ad sodium hydroxide, and dosing is performed by controlling the chemical pumps 28. As an example of an appropriate microprocessor for use in the system the CISC microcontroller, SAB80C535 of Siemens, may be mentioned. However, it will be possible to use other microprocessors. The microprocessor 26 is connected with a display unit. Values expressing the quality of the purified water and the present chemical consumption may be read into the display. Other parameters may also be displayed if desired.

It should be noted that the method of adjustment and the apparatus according to the present invention may also be used in combination with methods and apparatuses for fluid separation such as described in European Patent Application No. 91915413.8. When the method/apparatus is used in combination with such plants, it is also preferred for the measuring sondes 23 to be arranged in the outlet of the heat exchanger. The addition of acid and base may also in such a apparatus take place during the actual process by using feeding members controlled by signals from the computing and control unit 24.

## Claims

1. A method for adjusting a separation process in cleaning of waste water, preferably liquid manure, and wherein the concentrations of ammonia and acetic acid in the water fraction from the separation process are determined, and wherein said concentrations are used as parameters for adjusting the separation process so that the concentrations of ammonia and acetic acid are kept within predetermined limits, **characterised** in that a distillation is part of the separation process, that the concentrations are determined in the water fraction from the distillation step by measuring values of pH and conductivity in the distillate, that said values are converted into equivalent values of ammonia and acetic acid concentrations in the water fraction, and that these calculated values are used as parameters for controlling the addition of acid and base, which are reacted with ammonia and acetic acid for the neutralisation thereof.

2. A method according to claim 1, **characterised** in that mechanical vapour compression is used in the separation process.

3. A method according to claim 1 or 2, **characterised** in that acid and base are added in a amount that is exactly sufficient to react with the total calculated amount of ammonia and acetic acid for neutralisation of the entire contents thereof.

4. A method according to any one of the preceding claims, **characterised** in that the concentrations of acetic acid and ammonia in the water fraction are determined according to the following algorithms:

$$c_{NH_4} = \frac{S \cdot 10^{-pH} \left( \lambda_{H_3O^+} + \lambda_{CH_3COO^-} \right) - 10^{pH-14} \left( \lambda_{OH^-} - \lambda_{CH_3COO^-} \right)}{\left( 1 - \dfrac{1}{1 + 10^{pKs\text{-}NH4\text{-}pH}} \right) \left( \lambda_{NH_4^+} + \lambda_{CH_3COO^-} \right)}$$

$$c_{CH_3COOH} = \frac{S \cdot 10^{-pH} \left( \lambda_{H_3O^+} - \lambda_{NH_4^+} \right) - 10^{pH-14} \left( \lambda_{OH^-} + \lambda_{NH_4^+} \right)}{\left( \dfrac{1}{1 + 10^{pKs\text{-}CH_3COOH\text{-}pH}} \right) \left( \lambda_{CH_3COO^-} + \lambda_{NH_4^+} \right)}$$

wherein S is the conductivity and $\lambda$ is the molar conductivity of the individual ions.

5. A method according to any one of the preceding claims, **characterised** in that the calculations of the concentrations of ammonia and acetic acid are registered in an electronic computing and control unit, which simultaneously controls the addition of acid and base in a amount for neutralisation of all ammonia and all acetic acid.

6. A method according to any one of the preceding claims, **characterised** in that the separation process is performed in a thermally insulated cabinet, and that the values of pH and conductivity are measured outside of the cabinet.

7. An apparatus for use by a method according to any one of the preceding claims, said apparatus comprising heating members for boiling the manure, cooling members for cooling and condensing the formed vapour, and container members for receiving the condensed water fraction, **characterised** in that it comprises measuring as well as computing and control members for measuring conductivity and pH values in the water fraction as well as for converting the measured values into equivalent concentrations of ammonia and acetic acid, and dosing members for adding acid and base to the water fraction in an amount according to signals from the computing and control members.

8. An apparatus according to claim 7, **characterised** in that the heating and cooling members are provided in the form of a heat exchanger arranged in an evaporation tank, which comprises a top and a sump interconnected by a tube comprising a circulation pump for fluid circulation, and that a compressor is arranged between the sides of the heat exchanger to circulate the vapour, and that said heat exchanger, circulation pump and compressor form part of a mechanical vapour compression plant.

9. An apparatus according to claim 7 or 8, **characterised** in that it is arranged in an insulated cabinet with the measuring as well as computing and control members arranged outside the cabinet.

10. An apparatus according to claim 7, 8 or 9, **characterised** in that the computing and control members are designed to transmit a signal directly to dosing members that automatically dose acid and base as a result of those signals.

**Patentansprüche**

1. Verfahren zur Anpassung eines Trennprozesses beim Säubern von Abwasser, vorzugsweise von flüssigem Düngemittel, wobei die Konzentrationen von Ammoniak und Essigsäure in der Wasserfraktion aus dem Trennprozeß bestimmt werden und wobei die Konzentrationen als Parameter zur Anpassung des Trennprozesses verwendet werden, so daß die Konzentrationen von Ammoniak und Essigsäure innerhalb vorgegebener Grenzen gehalten werden, dadurch gekennzeichnet, daß eine Destillation Teil des Trennprozesses ist, daß die Konzentrationen in der Wasserfraktion aus der Destillationsstufe bestimmt werden, indem der pH-Wert und der Wert der Leitfähigkeit im Destillat gemessen werden, daß die Werte in äquivalente Werte der Ammoniak- und Essigsäurekonzentrationen in der Wasserfraktion umgewandelt werden und daß diese errechneten Werte als Parameter für die Steuerung der Zugabe von Säure und Base verwendet werden, welche mit Ammoniak und Essigsäure für die Neutralisation derselben zur Reaktion gebracht werden.

2. Verfahren nach Anspruch 1, dadurch gekennzeichnet, daß im Trennprozeß mechanische Dampfkompression verwendet wird.

3. Verfahren nach Anspruch 1 oder 2, dadurch gekennzeichnet, daß Säure und Base in einer Menge zugegeben werden, die genau ausreichend ist, um mit der gesamten errechneten Menge an Ammoniak und Essigsäure für die Neutralisation deren gesamter Anteile zu reagieren.

4. Verfahren nach mindestens einem der vorstehenden Ansprüche, dadurch gekennzeichnet, daß die Konzentrationen von Essigsäure und Ammoniak in der Wasserfraktion gemäß den folgenden Algorithmen bestimmt werden:

$$c_{NH_4} = \frac{S \cdot 10^{-pH} \, (\lambda_{H_3O^+} + \lambda_{CH_3COO^-}) - 10^{pH-14} \, (\lambda_{OH^-} - \lambda_{CH_3COO^-})}{\left(1 - \dfrac{1}{1 + 10^{pK_s\text{-}NH4\text{-}pH}}\right) \, \left(\lambda_{NH_4^+} + \lambda_{CH_3COO^-}\right)}$$

$$c_{CH_3COOH} = \frac{S \cdot 10^{-pH} \, \left(\lambda_{H_3O^+} - \lambda_{NH_4^+}\right) - 10^{pH-14} \left(\lambda_{OH^-} + \lambda_{NH_4^+}\right)}{\left(\dfrac{1}{1 + 10^{pK_s\text{-}CH_3COOH\text{-}pH}}\right) \, \left(\lambda_{CH_3COO^-} + \lambda_{NH_4^+}\right)}$$

wobei S die Leitfähigkeit und $\lambda$ die molare Leitfähigkeit der individuellen Ionen ist.

5. Verfahren nach mindestens einem der vorstehenden Ansprüche, dadurch gekennzeichnet, daß die errechneten Werte der Konzentrationen von Ammoniak und Essigsäure in einer elektronischen Computer- und Steuereinheit gespeichert werden, die gleichzeitig die Zugabe von Säure und Base in einer Menge für die Neutralisierung des gesamten Ammoniaks und der gesamten Essigsäure steuert.

6. Verfahren nach mindestens einem der vorstehenden Ansprüche, dadurch gekennzeichnet, daß der Trennprozeß in einem thermisch isolierten Gehäuse ausgeführt wird und daß der pH-Wert und der Wert der Leitfähigkeit außerhalb des Gehäuses gemessen werden.

**7.** Apparat für die Verwendung bei einem Verfahren nach einem der vorstehenden Ansprüche, wobei der Apparat Heizelemente zum Kochen des Düngemittels, Kühlelemente zum Kühlen und Kondensieren des gebildeten Dampfes und Behältermittel zum Aufnehmen der kondensierten Wasserfraktion aufweist, dadurch gekennzeichnet, daß er sowohl Meß- als auch Computer- und Steuerelemente zum Messen des Wertes der Leitfähigkeit und des pH-Wertes in der Wasserfraktion sowie zum Umwandeln der gemessenen Werte in äquivalente Konzentrationen von Ammoniak und Essigsäure und Dosierelemente, um Säure und Base der Wasserfraktion in einer Menge entsprechend den Signalen der Computer- und Kontrollelemente zuzugeben, aufweist.

**8.** Apparat nach Anspruch 7, dadurch gekennzeichnet, daß die Heiz- und Kühlelemente in Form eines Wärmetauschers vorgesehen sind, der in einem Verdampfertank angeordnet ist, welcher eine Spitze und einen Sumpf aufweist, die miteinander durch eine Röhre verbunden sind, welche eine Umwälzpumpe für die Fluidumwälzung aufweist, und daß ein Kompressor zwischen den Seiten des Wärmetauschers angeordnet ist, um den Dampf umzuwälzen, und daß der Wärmetauscher, die Umwälzpumpe und der Kompressor einen Teil einer mechanischen Dampfkompressionsanlage bilden.

**9.** Apparat nach Anspruch 7 oder 8, dadurch gekennzeichnet, daß er in einem Isolationsgehäuse angeordnet ist, wobei sowohl die Meß- als auch die Computer- und Steuerelemente außerhalb des Gehäuses angeordnet sind.

**10.** Apparat nach Anspruch 7, 8 oder 9, dadurch gekennzeichnet, daß die Computer- und Steuerelemente dafür ausgelegt sind, ein Signal direkt zu Dosierelementen hin zu übertragen, die als Folge jener Signale automatisch Säure und Base zudosieren.

## Revendications

**1.** Méthode pour ajuster un procédé de séparation dans l'épuration des eaux usées, de préférence de l'engrais liquide, et où les concentrations en ammoniaque et acide acétique dans la fraction d'eau du procédé de séparation sont déterminées, et où lesdites concentrations sont utilisées comme paramètres pour ajuster le procédé de séparation de manière que les concentrations d'ammoniaque et d'acide acétique soient maintenues dans des limites prédéterminées, caractérisée en ce qu'une distillation fait partie du procédé de séparation, en ce que les concentrations sont déterminées dans la fraction d'eau de l'étape de distillation en mesurant les valeurs du pH et la conductivité dans le distillat, en ce que lesdites valeurs sont converties en valeurs équivalentes de concentrations d'ammoniaque et d'acide acétique dans la fraction d'eau, et en ce que ces valeurs calculées sont utilisées comme paramètres pour contrôler l'addition de l'acide et de la base, que l'on fait réagir avec l'ammoniaque et l'acide acétique pour leur neutralisation.

**2.** Méthode selon la revendication 1, caractérisée en ce qu'une compression mécanique de vapeur est utilisée dans le procédé de séparation.

**3.** Méthode selon la revendication 1 ou 2, caractérisée en ce que l'acide et la base sont ajoutés en une quantité qui est exactement suffisante pour réagir avec la quantité totale calculée d'ammoniaque et d'acide acétique pour la neutralisation de toutes leurs teneurs.

**4.** Méthode selon l'une quelconque des revendications précédentes, caractérisée en ce que les concentrations en acide acétique et ammoniaque dans la fraction d'eau sont déterminées Selon les algorithmes suivants :

$$c_{NH_4} = \frac{S \cdot 10^{-pH} \, (\lambda_{H_3O^+} + \lambda_{CH_3COO^-}) \cdot 10^{pH-14} \, (\lambda_{OH^-} \cdot \lambda_{CH_3COO^-})}{\left(1 - \dfrac{1}{1 + 10^{pK_{s,NH4}-pH}}\right) \left(\lambda_{NH_4^+} + \lambda_{CH_3COO^-}\right)}$$

$$c_{CH_3COOH} = \frac{S \cdot 10^{-pH} \left(\lambda_{H_3O^+} \cdot \lambda_{NH_4^+}\right) \cdot 10^{pH-14} \left(\lambda_{OH^-} + \lambda_{NH_4^+}\right)}{\left(\dfrac{1}{1 + 10^{pK_{s,CH_3COOH}-pH}}\right) \left(\lambda_{CH_3COO^-} + \lambda_{NH_4^+}\right)}$$

où S est la conductivité et $\lambda$ est la conductivité molaire des ions individuels.

5. Méthode selon l'une quelconque des revendications précédentes, caractérisée en ce que les calculs de concentration d'ammoniaque et d'acide acétique sont enregistrés dans une unité électronique de calcul et de contrôle, qui contrôle simultanément l'addition d'acide et de la base en une quantité pour la neutralisation de toute l'ammoniaque et de tout l'acide acétique.

6. Méthode selon l'une quelconque des revendications précédentes, caractérisée en ce que le procédé de séparation est accompli dans un coffret thermiquement isolé, et en ce que les valeurs du pH et de la conductivité sont mesurées en dehors du coffret.

7. Appareil à utiliser par une méthode selon l'une quelconque des revendications précédentes, ledit appareil comprenant des éléments chauffants pour faire bouillir l'engrais, des éléments de refroidissement pour refroidir et condenser la vapeur formée, et des éléments conteneurs pour recevoir la fraction d'eau condensée, caractérisé en ce qu'il comprend des éléments de mesure ainsi que de calcul et de contrôle pour mesurer les valeurs de conductivité et du pH dans la fraction d'eau ainsi que pour convertir les valeurs mesurées en concentrations équivalentes d'ammoniaque et d'acide acétique, et des éléments de dosage pour ajouter l'acide et la base à la fraction d'eau en une quantité selon les signaux des éléments de calcul et de contrôle.

8. Appareil selon la revendication 7, caractérisé en ce que les éléments chauffants et le refroidissement sont prévus sous la forme d'un échangeur de chaleur agencé dans un réservoir d'évaporation, qui comprend un haut et une fosse interconnectés par un tube comprenant une pompe de circulation pour la circulation du fluide, et en ce qu'un compresseur est agencé entre les côtés de l'échangeur de chaleur pour faire circuler la vapeur, et en ce que ledit échangeur de chaleur, la pompe de circulation et le compresseur font partie d'une installation de compression mécanique de vapeur.

9. Appareil selon la revendication 7 ou 8, caractérisé en ce qu'il est agencé dans un coffret isolé avec les éléments de mesure ainsi que de calcul et de contrôle agencés en dehors du coffret.

10. Appareil selon la revendication 7, 8 ou 9, caractérisé en ce que les éléments de calcul et de contrôle sont conçus pour transmettre un signal directement à des éléments de dosage qui dosent automatiquement l'acide et la base par suite de ces signaux.

FIG.1

FIG. 2